Europäisches Patentamt

European Patent Office

Office européen des brevets

(19)

(11) Publication number: **0 394 479**
**A1**

## EUROPEAN PATENT APPLICATION
### published in accordance with Art. 158(3) EPC

(21) Application number: 89911107.4

(22) Date of filing: 06.10.89

(86) International application number:
PCT/JP89/01028

(87) International publication number:
WO 90/04031 (19.04.90 90/09)

(51) Int. Cl.⁵: **C12N 15/60, C12N 1/21, C12N 9/88**

(30) Priority: 06.10.88 JP 250869/88
09.12.88 JP 309868/88

(43) Date of publication of application:
31.10.90 Bulletin 90/44

(84) Designated Contracting States:
BE CH DE FR GB IT LI NL

(71) Applicant: **MITSUI TOATSU CHEMICALS, Inc.**
**2-5 Kasumigaseki 3-chome**
**Chiyoda-Ku Tokyo 100(JP)**

(72) Inventor: **ISHIWATA, Kenichi**
**314, Kamikuratacho, Totsuka-ku**
**Yokohama-shi, Kanagawa 244(JP)**
Inventor: **YOSHINO, Setsuo**
**2882, Iijimacho, Sakae-ku**
**Yokohama-shi, Kanagawa 244(JP)**
Inventor: **IWAMORI, Satoru**
**2070, Iijimacho, Sakae-ku**
**Yokohama-shi, Kanagawa 244(JP)**
Inventor: **FUKUOKA, Akio**
**3-4-24, Higashi 4 Jou Minami 12-chome**
**Sunagawa-shi, Hokkaido 073-01(JP)**
Inventor: **MAKIGUCHI, Nobuyoshi**
**2-12-23, Honkugenuma**
**Fujisawa-shi, Kanagawa 251(JP)**

(74) Representative: **Holdcroft, James Gerald, Dr.**
**et al**
**Graham Watt & Co., Riverhead**
**Sevenoaks, Kent TN13 2BN(GB)**

(54) GENE CODING FOR TYROSINE PHENOL-LYASE AND ITS UTILIZATION.

(57) A structural gene for tyrosine phenol-lyase has been cloned to clarify its DNA base sequence. *Escherichia coli* transformed by a recombinant plasmide having a structure wherein this structural gene is linked to the downstream part of tac promoter yields the tyrosine phenol-lyase in high productivity when cultured in an L-tyrosine-free medium.

TITLE MODIFIED
see front page

SPECIFICATION

## GENE CODING FOR TYROSINE PHENOL-LYASE AND USE THEREOF

Technical Field

This invention relates to a tyrosine phenol-lyase gene which is useful for the production of tyrosine phenol-lyase (EC 4.1.99.2), to recombinant plasmids containing said gene, to Escherichia coli strains transformed with said recombinant plasmids and to a process for the production of tyrosine phenol-lyase by using said E. coli strains.

Tyrosine phenol-lyase is a so-called multifunctional enzyme which catalyzes various reactions such as synthesis of L-tyrosine or L-dopa (Yamada, H. and Kumagai, H. (1975) Adv. Appl. Microbiol., Vol. 19, pp. 249-288) and is an industrially important enzyme.

Background Art

It is known that various bacteria have the tyrosine phenol-lyase activity. In particular, bacteria such as those of the genus Escherichia, the genus Citrobacter, the genus Aerobacter, the genus Proteus and the genus Erwinia, which belong to Enterobacteriaceae, are known to have high tyrosine phenol-lyase activities (Yamada, H. and Kumagai, H. (1975): Adv. Appl. Microbiol. Vol. 19, pp. 249-288).

Furthermore, DNA fragments containing the tyrosine phenol-lyase gene were isolated from strains of Erwinia herbicola ATCC-21434 and Escherichia freundii AJ-2608, and E. coli strains transformed with plasmids containing each of the DNA fragments are known to have tyrosine phenol-lyase activity (Japanese Patent Laid-Open Nos. 259589/1987 and 222682/1988).

However, the enzyme activity of these microorganisms was not sufficient for the industrial use, and furthermore, in the cultivation, it has been necessary to add tyrosine (Yamada, H. and Kumagai, H. (1975) Adv. Appl. Microbiol., Vol. 19, pp.249-288; Japanese Patent Laid-Open No. 259589/1987) and isopropyl-beta-thiogalactoside (Japanese Patent Laid Open No. 222682/1988) to a medium as an inducing agent. The addition of tyrosine to the medium caused great difficulty in removing remaining tyrosine in the production of amino acids other than tyrosine, e.g., dopa, and the addition of isopropyl-beta-thiogalactoside was very expensive, which limits their industrial use.

Disclosure of Invention

In the course of intensive study on the methods for the effective production of tyrosine phenol-lyase using microorganisms, the present inventors have isolated a DNA fragment which carries the tyrosine phenol-lyase gene derived from Citrobacter freundii, revealed the structure of the gene. Furthermore, they constructed, based on the analysis of the gene, a novel recombinant plasmid in which the structural gene for tyrosine phenol-lyase was linked to the downstream of the tac promoter and a novel E. coli strain transformed with the plasmid, found that this E. coli strain produces a large amount of tyrosine phenol-lyase without an addition of any inducing agent to the medium, and thus completed the present invention.

Namely, the present invention provides a useful gene for the production of tyrosine phenol-lyase without an addition of any inducing agent to a medium, a recombinant plasmid carrying the gene, a strain of E. coli transformed with the recombinant plasmid and a method for the production of tyrosine phenol-lyase using the E. coli transformant.

Brief Description of Drawings

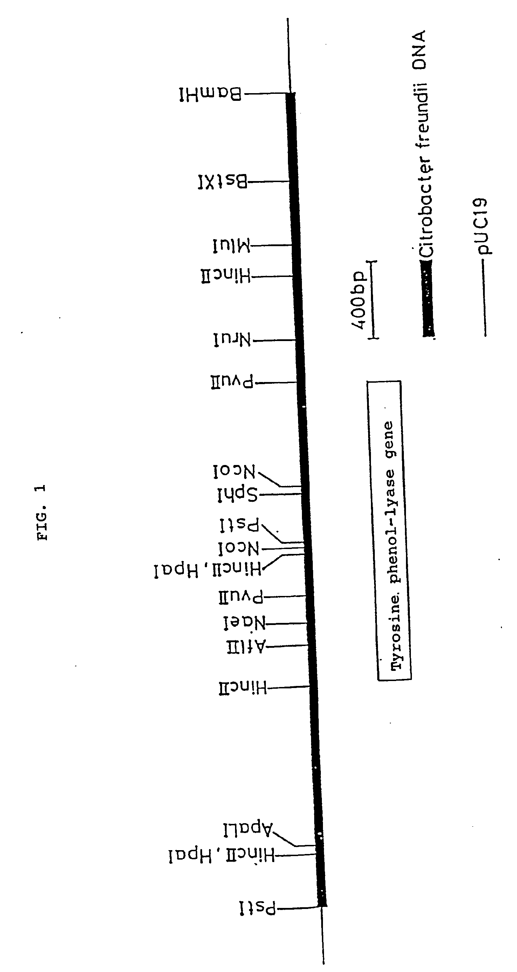

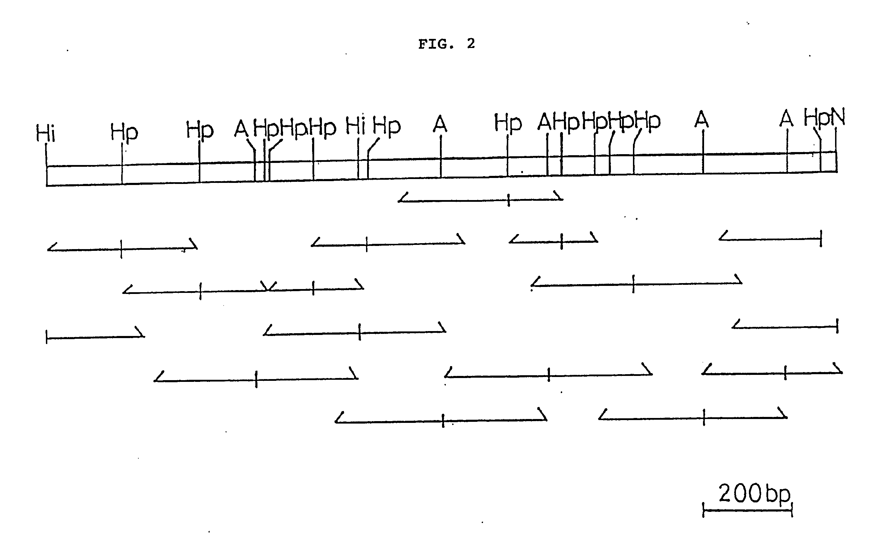

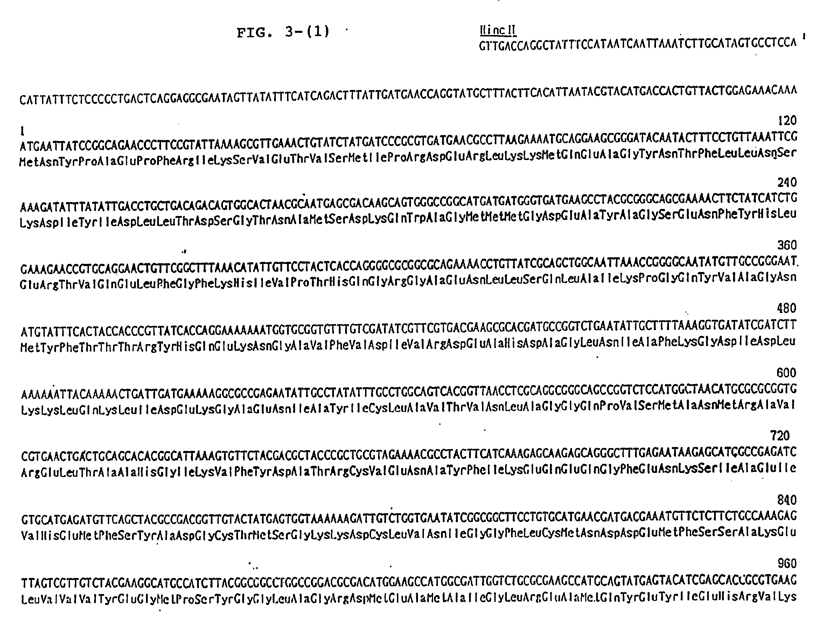

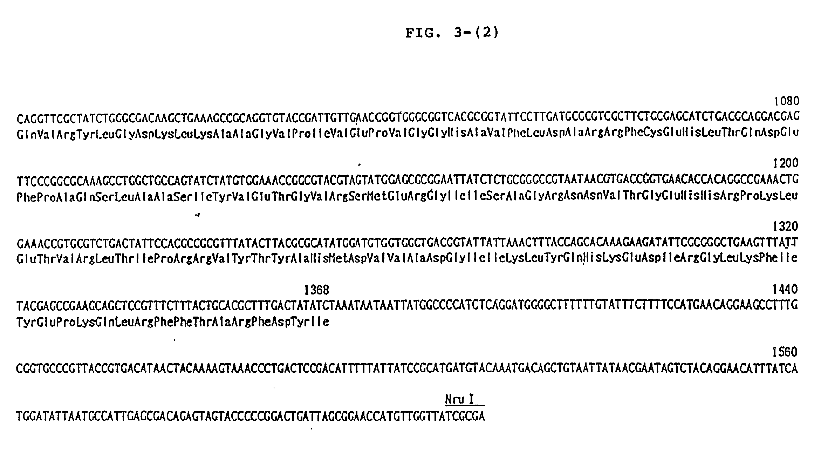

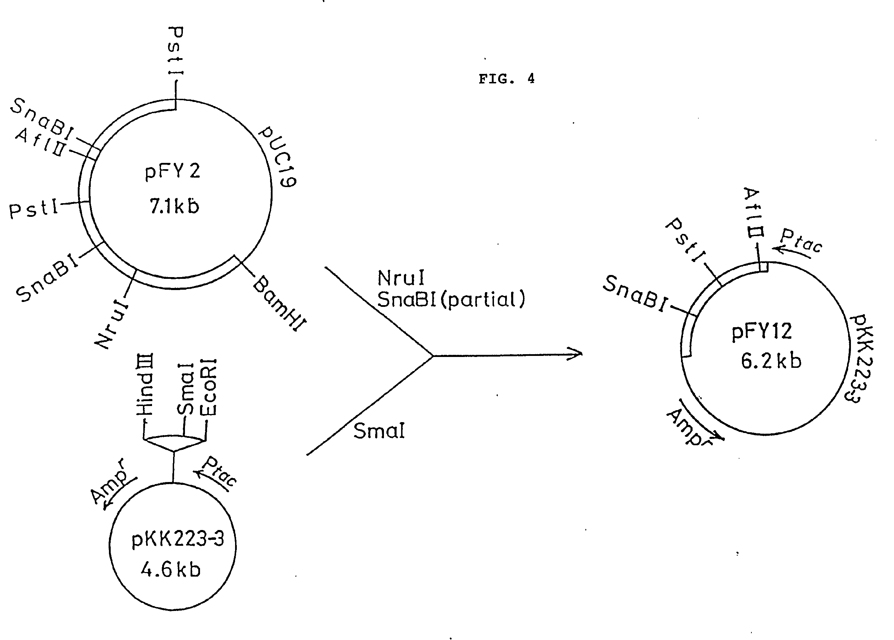

Fig. 1 shows a restriction endonuclease cleavage map of an insert DNA, derived from Citrobacter freundii and an approximate location of the tyrosine phenol-lyase gene in recombinant plasmid pFY2. Fig. 2 shows the principle in determination of a DNA base sequences according to the dideoxy method. Fig. 3 shows the DNA base sequence of the tyrosine phenol-lyase gene and the amino acid sequence of the tyrosine phenol-lyase. Fig. 4 shows the restriction endonuclease cleavage map for recombinant plasmid pFY12.

Best Mode for Carrying Out the Invention

According to the present invention, microorganisms which are used to take a DNA segment carrying a tyrosine phenol-lyase gene are those having tyrosine phenol-lyase activity, for example Citrobacter freundii MT-10419 (FERM BP-2624: FERM P-10181).

Methods for taking a DNA segment carrying a tyrosine phenol-lyase gene is not particularly specified. For example, the following methods may be used:

A microorganism having tyrosine phenol-lyase activity is cultured, and then chromosomal DNA is then extracted from the resultant cells, for example by the phenol method (Saito, H. et al. (1963) Biochim. Biophys. Acta., Vol. 72, pp. 619-629) and purified. The chromosomal DNA is cleaved with an appropriate restriction endonuclease and enzymatically ligated using DNA ligase to a plasmid vector which is replicable in E. coli and then cells of E. coli lacking the capability for producing tyrosine phenol-lyase are transformed using the resultant recombinant DNA. The resultant strain which obtained the capability for producing tyrosine phenol-lyase are selected, and then the plasmid DNA carrying the tyrosine phenol-lyase gene can be isolated from this strain.

In the present invention, various kinds of restriction endonucleases for cleaving chromosomal DNA can be used by controlling the amount of the endonucleases to be used and the reaction time so as to control the extent of the DNA cleavage. For example, HpaII, AccI, Sau3AI, HpaI or ScaI are used.

As for the plasmid vector, one which is capable of replicating in E. coli is used; for example, a plasmid of ColEl line such as pBR322 or pUC19 is used.

The plasmid vector can be ligated to a chromosomal DNA fragment using DNA ligase after being cleaved by the same restriction endonuclease as used in the cleavage of chromosomal DNA or a restriction endonuclease that can generate the identical cohesive ends as said restriction endonuclease generates.

As for DNA ligase, one derived from T4 phage is preferably used.

After the cleavage by restriction endonuclease, chromosomal DNA may be fractionated and recovered by sucrose density gradient centrifugation, agarose electrophoresis or the like so as to obtain a DNA fragment having a specified size only.

In order to introduce the resultant recombinant DNA comprising the chromosomal DNA fragment and the plasmid vector into E. coli, the method in which bacterial cells are treated with calcium chloride (Mandel, O. et al. (1970) J. Mol. Biol., Vol. 53, pp. 159-162) or the like can be used. As for an E. coli strain which lacks the capability for producing tyrosine phenol-lyase, E. coli MC-1061, JM-83 or the like can be used.

For the selection of transformants which gained the capability for producing tyrosine phenol-lyase, the following method, for example, can be used:

Colonies of transformants are formed on an agar plate of L-medium supplemented with tyrosine. Then 4-aminoantipyrine is sprayed on this agar plate and transformants which form colonies with the red circumsference may be selected.

In order to isolate a recombinant plasmid from E. coli cells having the recombinant plasmid, the alkali extraction method (Brinboim, H. et al. (1979) Nucleic Acids Res., Vol. 7, pp. 1513-1523) or the like can be used.

The recombinant plasmid thus isolated can be introduced again into E. coli cells by the method in which the cells are treated with calcium chloride (Mandel, O. et al. (1970) J. Mol. Biol., Vol. 53, pp. 619-629) or the like.

pFY2 is an example of the recombinant plasmid obtained as described above, in which DNA carrying tyrosine phenol-lyase gene is ligated to a plasmid vector.

The following strain has been deposited at the Fermentation Research Institute, Agency of Industrial Science and Technology as an E. coli strain carrying pFY2:

Escherichia coli MT-10473 (MC-1061/pFY2) (FERM BP-2620: alteration of FERM P-10182)

In the present invention, in order to efficiently express tyrosine phenol-lyase gene without adding any inducing agent to a medium, a region of tyrosine phenol-lyase gene is cut from pFY2 and ligated to a vector having a strong promoter downstream of the promoter. As the promoter, the tac promoter is preferably used. Furthermore, in some cases, ligation of a terminator downstream of the tyrosine phenol-lyase gene is effective. An example of the recombinant plasmid described above is pFY12.

The following strain has been deposited at the Fermentation Research Institute, Agency of Industrial Science and Technology as an E. coli strain carrying pFY12:

Escherichia coli MT-10488 (MC-1061/pFY12) (FERM BP-2616)

In the present invention, in order to produce tyrosine phenol-lyase, E. coli cells transformed with the recombinant plasmid thus obtained may be cultured to produce tyrosine phenol-lyase in a medium.

Cultivation of E. coli is carried out by an ordinary method. Namely, the cultivation may be carried out aerobically using a medium containing a carbon source, a nitrogen source, inorganic ions, and furthermore, if required, amino acids and vitamins. Furthermore, in the present invention, it is not necessary to add any inducing agent to the medium, such as tyrosine, and isopropyl-beta thiogalactoside.

The culture thus obtained can be used as an enzyme source of tyrosine phenol-lyase at it is; however, a crude enzyme extract, a purified enzyme preparation, isolated living bacterial cells and a treated bacterial cells are also used as the enzyme source.

As the method for the purification of tyrosine phenol-lyase, an ordinary process for enzyme purification can be used.

[Example]

The present invention will be explained more in detail by the following Example:

Example 1

(a) Isolation of chromosomal DNA of Citrobacter freundii

Cells of Citrobacter freundii MT-10419 (a strain deposited at the Fermentation Research Institute, Agency of Industrial Science and Technology on August 6, 1985 for the in-Japan deposition (deposition number: FERMP-10181) (later deposited under the Budapest Treaty with an altered deposition number, FERM BP-2624) were inoculated into a 1 l of L medium (10g/l Bacto Tryptone, 5g/l yeast extract, 5g/l sodium chloride; pH was adjusted to 7.2), and after cultivation at 30°C for 15 hours with shaking, the cells were harvested by centrifugation.

The cells were suspended in 160 ml of a 0.15M NaCl-50mM EDTA (pH 8.0) solution, and the suspension was gently stirred with an addition of 160 mg of lysozyme at 37°C for 20 minutes. To the suspension was then added 4 ml of a 20 % SDS solution and the suspension was allowed to stand at 65°C for 20 minutes. Furthermore, to the suspension was added 8 mg of proteinase K (a product of Boehringer Mannheim), and the suspension was allowed to stand at 37°C for 1 hours.

To the suspension was added 160 ml of phenol saturated with a 0.15M NaCl-50mM EDTA (pH 8.0) solution, gently stirred and then centrifuged at 10,000 rpm for 15 minutes to recover the upper layer fraction.

To the solution thus obtained was added a 2-fold volume of cold ethanol, and the fiber-like precipitate was recovered by winding it round a glass rod, immersed in 70%, 80% and 90% ethanol in order each for several minutes, dried and then dissolved in 40 ml of a 0.1M NaCl-0.15M sodium citrate solution (pH 7.0).

To this crude DNA solution was added 200 microliter of ribonuclease A (6 mg/ml, a product of Boehringer Mannheim) and the solution was allowed to stand at 37°C for 1.5 hours.

To this solution was added 40 ml of phenol saturated with a 0.15M NaCl-50mM EDTA solution (pH 8.0), followed by gentle stirring, and the resulting mixture was centrifuged at 10,000 rpm for 15 minutes to recover the upper layer.

To this upper layer was added a 2-fold volume of cold ethanol, and the fiber-like precipitate was recovered by winding it round a glass rod, immersed in 70%, 80% and 90% ethanol in order each for several minutes, dried and then dissolved in 20 ml of a 10mM Tris-HCl (pH8.0)-1mM EDTA solution (hereinafter referred to as the TE buffer solution).

This DNA solution was dialyzed against 2 l of the TE buffer solution and then 10 ml of the TE buffer solution containing 2 mg of chromosomal DNA was obtained.

(b) Purification of chromosomal DNA fragment

To a 450-microliter portion (containing 90 micrograms of DNA) of the TE buffer solution containing chromosomal DNA obtained in section (a) was added 10 units of the restriction endonuclease HpaII (a product of Boehringer Mannheim) and the resultant solution was allowed to stand in 500 microliters of a reaction solution containing 10 mM Tris-HCl (pH 7.5), 7 mM $MgCl_2$ and 7 mM 2-mercaptoethanol at $37^{\circ}C$ for 1 hour to partially digest the chromosomal DNA.

To this solution was added an equal volume of a phenol-chloroform mixture (phenol:chloroform = 1:1) saturated with the TE buffer solution, followed by gentle stirring, and the mixture was centrifuged at 15,000 rpm for 5 minutes to recover the upper layer.

To the upper layer thus recovered was added a 2-

fold volume of cold ethanol, and the resultant precipitate was dried and then dissolved in 100 microliters of the TE buffer solution. This DNA solution was subjected to 10-40 % sucrose density gradient centrifugation (at 20°C at 26,000 rpm for 24 hours) and thus two fractions, about 2-5 kbp and 5-10 kbp in size, were recovered. Each fraction was dialyzed against the TE buffer solution, and the DNA was then recovered by ethanol precipitation and dissolved in the TE buffer solution.

(c) Ligation of chromosomal DNA fragment to plasmid vector

Ten micrograms of pUC 19 (a product of Takara Shuzo K.K.) was completely digested with the restriction enzyme AccI (a product of Boehringer Mannheim) and then treated with alkaline phosphatase.

Each fraction of the chromosomal DNA fragment prepared in section (b) was mixed with 0.25 microgram each of this plasmid vector, and each mixture was reacted in 0.4 ml of a reaction solution (5 mM $MgCl_2$, 10 mM dithiothreitol, 1 mM ATP and a 66 mM Tris-HCl buffer solution (pH 7.5)) using 5 units of T4 ligase (a product of Boehringer Mannheim) at 16°C for 16 hours.

For transformation of E. coli, this reaction solution was used as it is.

(d) Cloning of tyrosine phenol-lyase gene

Transformation of E. coli was carried out using the reaction mixture containing the recombinant plasmid prepared in section (c).

Cells of E. coli MC-1061 strain lacking the capability of producing tyrosine phenol-lyase were inoculated into 5 ml of an L medium (10g/l Bacto Tryptone, 5g/l yeast extract and 5g/l sodium chloride; pH adjusted to 7.2), and the cells were cultivated with shaking at $37^{\circ}C$ for 3 hours and then harvested by centrifugation.

The cells were suspended in 2 ml of a 0.1 M $CaCl_2$ solution, allowed to stand at $0^{\circ}C$ for 30 minutes, centrifuged and resuspended in 4 ml of a 0.1 M $CaCl_2$ solution.

Two milliliters each of the cell suspension thus prepared was dispensed into two test tubes, allowed to stand at $0^{\circ}C$ for 3 hours after adding the reaction solutions prepared in section (c) to the test tubes, respectively, and then maintained at $42^{\circ}C$ for 2 minutes.

The cells were harvested by centrifugation, suspended in 5 ml each of the L medium and then incubated at $37^{\circ}C$ for 30 minutes; the resultant cells were harvested by centrifugation.

The cells were suspended in 10 ml each of 0.85% NaCl, collected by centrifugation and then resuspended in 1 ml each of 0.85% NaCl.

The cell suspensions thus prepared were plated on the L mediums supplemented with 100mg/l ampicillin, 20g/l L-tyrosine and 15g/l agar and incubated at $37^{\circ}C$ for 1 day.

Subsequently, aqueous solutions of (1) 1.4 % sodium hydrogencarbonate, (2) 0.85% 4-aminoantipyrine and (3) 5.4% potassium ferricyanide were sprayed in order on each of the plate, and then strains which formed colonies with the red circumsference were selected.

From the agar plate medium spread with E. coli cells transformed with the recombinant plasmid prepared using a fraction of chromosomal DNA fragment of 2-5kbp in size, three tyrosine phenol-lyase producing strains were obtained. Among them, an E. coli strain isolated from the representative colony has been named Escherichia coli MT-10473 and deposited at the Fermentation Research Institute of the Agency of Industrial Science and Technology on August 6, 1988 (deposit number: FERM P-10182). Further, this deposition was altered to the international deposition under the Budapest Treaty and the altered number is FERM BP-2620.

(e) Isolation of recombinant plasmid retained in E. coli

A recombinant plasmid was isolated from Escherichia coli MT-10473 by the following method:

Cells of Escherichia coli MT-10473 were inoculated in 100 ml of the L medium and incubated with shaking at $37^{O}C$ for 15 hours and then the resultant cells were harvested by centrifugation.

The cells were suspended in 4 ml of a 50mM glucose-25mM Tris-HCl-10mM EDTA (pH 8.0) solution containing 4 mg of lysozyme.

To the solution was added 8 ml of a 0.2N NaOH-1% SDS solution, followed by stirring. Six milliliters of a 3M $CH_3COONa$ (pH 5.2) solution was added thereto, and the resultant solution was allowed to stand at $4^0C$ for 5 minutes and then centrifuged to recover the supernatant.

To this supernatant solution was added a phenol-chloroform mixture (phenol:chloroform = 1:1) saturated with the TE buffer solution, and the mixture was gently stirred and then centrifuged at 10,000 rpm for 5 minutes to recover the upper layer.

To the upper layer thus recovered was added a 2-fold volume of cold ethanol, and the precipitate formed was dried and then dissolved in 1ml of the TE buffer solution.

To this DNA solution was added 20 microliters of ribonuclease A (1mg/ml, a product of Boehringer Mannheim), and the resultant solution was allowed to stand at $37^0C$ for 20 minutes.

To this solution was added an equal volume of a phenol-chloroform mixture (phenol:chloroform = 1:1), and the mixture was stirred gently and then centrifuged at 10,000 rpm for 5 minutes to recover the upper layer.

This DNA solution was purified by column chromatography using Bio-Gel A-50m (a product of Bio-Rad) and plasmid DNA was recovered by ethanol precipitation.

About 1.2 mg of the plasmid DNA was thus isolated from Escherichia coli MT-10473 and suspended in 1ml of the TE buffer solution.

The plasmids obtained as described above was used in the analysis of the recombinant plasmid as described below.

(f) Analysis of recombinant plasmid

The recombinant plasmid isolated from <u>Escherichia</u> <u>coli</u> MT-10473 was named pFY2. This recombinant plasmid was about 7.1 kbp in size and when cleaved with the restriction endonucleases EcoRI and HindIII, a DNA insert fragment of about 4.4 kbp was observed.

The restriction endonuclease cleavage map of the insert DNA fragment in pFY2 is shown in Fig. 1.

(g) Confirmation of reproducibility of transformation

Cells of <u>E. coli</u> MC-1061 strain lacking the capability for producing tyrosine phenol-lyase were transformed in the same manner as in section (d) with the recombinant plasmid pFY2 isolated in section (e) and then plated on the L medium agar plate supplemented with 100mg/l ampicillin and 20g/l L-tyrosine. Examination of the formed colonies for tyrosine phenol-lyase production by the 4-aminoantipyrine method as described in section (d) revealed that the circumsference of all the colonies was colored red, which confirmed the presence of DNA carrying the tyrosine phenol-lyase gene.

(h) Subcloning and analysis of DNA fragment

Various DNA fragments lacking a part of the inserted DNA fragment of pFY2 were prepared and inserted individually into a polylinker region downstream of the tac promoter of the expression vector pKK223-3 (a product of Pharmacia) using lyase. With these recombinant plasmids cells of Escherichia coli MT-1061 were transformed as described in section (d) and then plated on an L medium agar plate supplemented with 100mg/l ampicillin and 20g/l L-tyrosine. Formed colonies were examined for tyrosine phenol-lyase production by the 4-aminoantipyrine method as described in section (d) and thereby the approximate position of tyrosine phenol-lyase gene, as shown in Fig. 1, was determined. In pFY2, the HincII-NruI insert DNA fragment of about 1.8 kb which was considered to contain the tyrosine phenol-lyase gene was subjected to the DNA base sequence determination by the dideoxy method (Sanger, F. et al. (1977) Proc. Natl. Acad. Sci. USA, Vol. 74, pp. 5463-5467) using a vector of the M13mp line (Messing, J. (1983) Method in Enzymology, Vol. 101, pp. 20-78) according to the principle shown in Fig. 2. As a result, the presence of an open reading frame as shown in Fig. 3 was confirmed in this base sequence.

(i) Construction of non-inducing plasmid for high expression of tyrosine phenol-lyase

One microgram of the plasmid pKK223-3 having the tac promoter (a product of Pharmacia) was completely digested with the restriction endonuclease SmaI (a product of New England Biolabs GmbH), treated with alkaline phosphatase and then recovered by ethanol precipitation to prepare vector DNA.

Separately, the plasmid pFY2 was extracted as described in section (e) and 2 micrograms thereof was completely digested with the restriction endonuclease NruI (a product of New England Biolabs GmbH) and then partly digested in a reaction solution (1 mM $MgCl_2$, 7 mM 2-mercaptoethanol, 50 mM NaCl, 0.01 % BSA and a 10 mM Tris-HCl buffer solution (pH 7.5)) with 1 unit of the restriction endonuclease SnaBI (a product of New England Biolabs GmbH) at $37^{o}C$ for 30 minutes. Then the DNA in the reaction solution was subjected to 0.8% agarose gel electrophoresis and a gel containing DNA of about 1.7 kb in size was cut off; DNA was recovered by electrical elution and ethanol precipitation and thereby the insert DNA containing the tyrosine phenol-lyase gene was prepared.

The vector DNA dissolved in the TE buffer solution and the insert DNA containing the tyrosine phenol-lyase gene were mixed and reacted in 0.1 ml of a reaction solution (5 mM $MgCl_2$, 10 mM dithiothreitol, 1 mM ATP and a 66 mM Tris-HCl buffer solution (pH 7.5)) using 300 units of T4 ligase (a product of Boehringer Mannheim) at $16^{o}C$ for 4 hours. This reaction solution itself was used to transform cells of E. coli MC-1061 lacking the capability of producing tyrosine phenol-lyase in the same manner as described in section (d), and the transformed cells were spread on an L medium agar plate supplemented with 100 mg/l ampicillin and 20g/l L-tyrosine. Colonies formed were examined for tyrosine phenol-lyase production by the 4-aminoantipyrine method as described in section (d), and, as a result, the tyrosine phenol-lyase production was observed in about 1000 strains. Four strains were selected from the transformants thus obtained and plasmids were extracted according to the method shown in section (e). All the plasmids obtained were about 6.2 kb in size and had an identical restriction endonuclease cleavage map. These plasmid were named pFY12. The restriction endonuclease map of pFY12 is shown in Fig. 4. A strain of E. coli MC-1061 transformed with pFY12 was named Escherichia coli MT-10488 and deposited at the Fermentation Research Institute, Agency of Industrial Science and Technology (FERM BP-2616).

(j) Production of tyrosine phenol-lyase

Cells of Escherichia coli MT-10488 were inoculated in 100 ml of an L medium (10g/l Bacto-Tryptone, 5g/l yeast extract and 5g/l sodium chloride; pH adjusted to 7.2) supplemented with 0 or 20g/l L-tyrosine, and after cultivation at 37°C for 20 hours with shaking the resultant cells were collected by centrifugation.

Tyrosine phenol-lyase activity of these cells was determined as follows:

The cells were washed and then suspended in 10 ml of a 10 mM pyrophosphate buffer solution (pH 8.5) containing 0.1 mM pyridoxal phosphate. Cell-free extract was prepared by ultrasonic treatment, and this enzyme solution was added to 90 ml of a reaction solution containing 70g/l L-serine, 17g/l phenol and 17g/l ammonium acetate (pH adjusted to 8.5 with ammonium), and the resulting mixture was gently stirred at 37°C for 1 hour for reaction. The amount of L-tyrosine produced was determined by the method of Kondo et al. (Kondo, N. et al. (1984) Agaric. Biol. Chem., Vol. 48, pp. 1595-1601) using high performance liquid chromatography. For comparison, cells of Escherichia coli MC-1061 used as a host, Citrobacter freundii MT-10419 used as a chromosomal DNA source and Escherichia coli MT-10473 carrying pFY2 were cultivated in the same manner and the tyrosine phenol-lyase activity was determined in the same manner.

Tyrosine phenol-lyase activity of each strains is shown in Table 1. Further, one unit of tyrosine phenol-lyase corresponds to the amount of enzyme which produces one micromole of tyrosine at 37°C in one minute.

In the case of the MT-10419 strain, the enzyme activity was observed only when L-tyrosine was added to the medium; furthermore in the case of the MT-10473 strain, the enzyme activity was observed even in the medium without an addition of L-tyrosine but the value was about 1/4 that observed in the medium with L-tyrosine added.

On the other hand, in the case of the MT-10488 strain, high enzyme activity was observed without an addition of L-tyrosine to the medium.

Table 1

| Strain | Tyrosine added to the medium (g/l) | Enzyme activity (Unit/g wet-cells) |
|--------|-----------------------------------|-----------------------------------|
| MT-10488 | 0 | 66.9 |
|          | 20 | 73.5 |
| MC-1061 | 0 | 0 |
|         | 20 | 0 |
| MT-10419 | 0 | 0 |
|          | 20 | 6.4 |
| MT-10473 | 0 | 5.9 |
|          | 20 | 25.0 |

Industrial Applicability

According to the present invention, a gene useful for the effective production of tyrosine phenol-lyase, a recombinant plasmid carrying the gene, E. coli transformed with the recombinant plasmid and a method for producing tyrosine phenol-lyase using the E. coli are provided.

Reference to the Deposition of Microorganisms

1. Escherichia coli MT-10419

Place of deposition

Name: The Fermentation Research Institute, Agency of Industrial Science and Technology

Address: 1-3 Higashi 1-chome, Tsukuba City, Ibaraki Prefecture, 305 Japan

Date of deposition: August 6, 1988

Deposition number: FERM BP-2624

[Deposition under the Budapest Treaty: Alteration of the corresponding in-Japan deposition (deposition number: FERM P-10181)]

2. Escherichia coli MT-10473

Place of deposition

Name: The Fermentation Research Institute, Agency of Industrial Science and Technology

Address: 1-3 Higashi 1-chome, Tsukuba City, Ibaraki Prefecture, 305 Japan

Date of deposition: August 6, 1988

Deposition number: FERM BP-2620

[Deposition under the Budapest Treaty: Alteration of the corresponding in-Japan deposition (deposition number: FERM P-10182)]

3. Escherichia coli MT-10488

Place of deposition

Name: The Fermentation Research Institute, Agency of Industrial Science and Technology

Address: 1-3 Higashi 1-chome, Tsukuba City, Ibaraki Prefecture, 305 Japan

Date of deposition: September 27, 1989

Deposition number: FERM BP-2616

(Deposition under the Budapest Treaty)

Claims

1.    A gene coding for tyrosine phenol-lyase represented by the following amino acid sequence:

```
MNYPAEPFRI   KSVETVSMIP   RDERLKKMQE   AGYNTFLLNS
KDIYIDLLTD   SGTNAMSDKQ   WAGMMMGDEA   YAGSENFYHL
ERTVQELFGF   KHIVPTHQGR   GAENLLSQLA   IKPGQYVAGN
MYFTTTRYHQ   EKNGAVFVDI   VRDEAHDAGL   NIAFKGDIDL
KKLQKLIDEK   GAENIAYICL   AVTVNLAGGQ   PVSMANMRAV
RELTAAHGIK   VFYDATRCVE   NAYFIKEQEQ   GFENKSIAEI
VHEMFSYADG   CTMSGKKDCL   VNIGGFLCMN   DDEMFSSAKE
LVVVYEGMPS   YGGLAGRDME   AMAIGLREAM   QYEYIEHRVK
QVRYLGDKLK   AAGVPIVEPV   GGHAVFLDAR   RFCEHLTQDE
FPAQSLAASI   YVETGVRSME   RGIISAGRNN   VTGEHHRPKL
ETVRLTIPRR   VYTYAHMDVV   ADGIIKLYQH   KEDIRGLKFI
YEPKQLRFFT   ARFDYI
```

(in which one-letter symbols are A: Alanine, C: Cysteine, D: Aspartic acid, E: Glutamic acid, F: Phenylalanine, G: Glycine, H: Histidine, I: Isoleucine, K: Lysine, L: Leucine, M: Methionine, N: Asparagine, P: Proline, Q: Glutamine, R: Arginine, S: Serine, T: Threonine, V: Valine, W: Tryptophan and Y: Tyrosine).

2.    A tyrosine phenol-lyase gene represented by the following DNA base sequence:

```
ATGAATTATC   CGGCAGAACC   CTTCCGTATT   AAAAGCGTTG

AAACTGTATC   TATGATCCCG   CGTGATGAAC   GCCTTAAGAA

AATGCAGGAA   GCGGGATACA   ATACTTTCCT   GTTAAATTCG

AAAGATATTT   ATATTGACCT   GCTGACAGAC   AGTGGCACTA

ACGCAATGAG   CGACAAGCAG   TGGGCCGGCA   TGATGATGGG

TGATGAAGCC   TACGCGGGCA   GCGAAAACTT   CTATCATCTG

GAAAGAACCG   TGCAGGAACT   GTTCGGCTTT   AAACATATTG

TTCCTACTCA   CCAGGGGCGC   GGCGCAGAAA   ACCTGTTATC

GCAGCTGGCA   ATTAAACCGG   GGCAATATGT   TGCCGGGAAT

ATGTATTTCA   CTACCACCCG   TTATCACCAG   GAAAAAAATG

GTGCGGTGTT   TGTCGATATC   GTTCGTGACG   AAGCGCACGA

TGCCGGTCTG   AATATTGCTT   TTAAAGGTGA   TATCGATCTT

AAAAAATTAC   AAAAACTGAT   TGATGAAAAA   GGCGCCGAGA

ATATTGCCTA   TATTTGCCTG   GCAGTCACGG   TTAACCTCGC

AGGCGGGCAG   CCGGTCTCCA   TGGCTAACAT   GCGCGCGGTG

CGTGAACTGA   CTGCAGCACA   CGGCATTAAA   GTGTTCTACG

ACGCTACCCG   CTGCGTAGAA   AACGCCTACT   TCATCAAAGA

GCAAGAGCAG   GGCTTTGAGA   ATAAGAGCAT   CGCCGAGATC

GTGCATGAGA   TGTTCAGCTA   CGCCGACGGT   TGTACTATGA

GTGGTAAAAA   AGATTGTCTG   GTGAATATCG   GCGGCTTCCT

GTGCATGAAC   GATGACGAAA   TGTTCTCTTC   TGCCAAAGAG

TTAGTCGTTG   TCTACGAAGG   CATGCCATCT   TACGGCGGCC

TGGCCGGACG   CGACATGGAA   GCCATGGCGA   TTGGTCTGCG
```

```
CGAAGCCATG   CAGTATGAGT   ACATCGAGCA   CCGCGTGAAG

CAGGTTCGCT   ATCTGGGCGA   CAAGCTGAAA   GCCGCAGGTG

TACCGATTGT   TGAACCGGTG   GGCGGTCACG   CGGTATTCCT

TGATGCGCGT   CGCTTCTGCG   AGCATCTGAC   GCAGGACGAG

TTCCCGGCGC   AAAGCCTGGC   TGCCAGTATC   TATGTGGAAA

CCGGCGTACC   TAGTATGGAG   CGCGGAATTA   TCTCTGCGGG

CCGTAATAAC   GTGACCGGTG   AACACCACAG   GCCGAAACTG

GAAACCGTGC   GTCTGACTAT   TCCACGCCGC   GTTTATACTT

ACGCGCATAT   GGATGTGGTG   GCTGACGGTA   TTATTAAACT

TTACCAGCAC   AAAGAAGATA   TTCGCGGGCT   GAAGTTTATT

TACGAGCCGA   AGCAGCTCCG   TTTCTTTACT   GCACGCTTTG

ACTATATC
```

3.    A recombinant plasmid in which DNA carrying the gene as set forth in claim 1 or 2 is linked to a plasmid vector.

4.    A recombinant plasmid as set forth in claim 3, in which DNA carrying the gene as set forth in claim 1 or 2 is linked downstream of a promoter.

5.    A recombinant plasmid as set forth in claim 4, in which said promoter is the tac promoter.

6.    A novel recombinant plasmid pFY12.

7.    Escherichia coli transformed with the recombinant plasmid as set forth in claims 3, 4, 5 or 6.

8.    A novel _Escherichia_ _coli_ MT-10488 strain.

9.    A method for production of tyrosine phenol-lyase, characterized in that cells of _Escherichia_ _coli_ as set forth in claim 7 or 8 are cultured and tyrosine phenol-lyase is thereby produced in the culture.

FIG. 1

Tyrosine. phenol-lyase gene

400bp

▬▬ Citrobacter freundii DNA

—— pUC19

FIG. 2

200 bp

EP 0 394 479 A1

FIG. 3-(1)

HincII
GTTGACCAGGCTATTTCCATAATCAATTAAATCTTGCATAGTGCCTCCA '

CATTATTTCTCCCCCTGACTCAGGAGGCCGAATAGTTATATTTCATCAGACTTTATTGATGAACCAGGTATGCTTTACTTCACATTAATACGTACATGACCACTGTTACTGGAGAAACAAA

ATGAATTATCCCGCAGAACCCTTCCGTATTAAAAGCGTTGAAACTGTATCTATGATCCCGCGTGATGAACGCCTTAAGAAAATGCAGGAAGCGGGATACAATACTTTCCTGTTAAAATTCG
MetAsnTyrProAlaGluProPheArgIleLysSerValGluThrValSerMetIleProArgAspGluArgLeuLysLysMetGlnGluAlaGlyTyrAsnThrPheLeuLeuAsnSer

AAAGATATTTATATTGACCTGCTGACAGACAGTGGCACTAACGCAATGAGCGACAAGCAGTGGGCCGGCATGATGATGGGTGATGAAGCCTACGCGGGCAGCGAAAACTTCTATCATCTG
LysAspIleTyrIleAspLeuLeuThrAspSerGlyThrAsnAlaMetSerAspLysGlnTrpAlaGlyMetMetMetGlyAspGluAlaTyrAlaGlySerGluAsnPheTyrHisLeu

GAAAGAACCGTGCAGGAACTGTTCGGCTTTAAACATATTGTTCCTACTCACCAGGGGCGCGGCGCAGAAAACCTGTTATCGCAGCTGGCAATTAAACCGGGGCAATATGTTGCCGGGAAT
GluArgThrValGlnGluLeuPheGlyPheLysHisIleValProThrHisGlnGlyArgGlyAlaGluAsnLeuLeuSerGlnLeuAlaIleLysProGlyGlnTyrValAlaGlyAsn

ATGTATTTCACTACCACCCGTTATCACCAGGAAAAAAATGGTGCGGTGTTTGTCGATATCGTTCGTGACGAAGCGCACGATGCCGGTCTGAATATTGCTTTTAAAGGTGATATCGATCTT
MetTyrPheThrThrThrArgTyrHisGlnGluLysAsnGlyAlaValPheValAspIleValArgAspGluAlaHisAspAlaGlyLeuAsnIleAlaPheLysGlyAspIleAspLeu

AAAAAATTACAAAAACTGATTGATGAAAAAGGCGCCGAGAATATTGCCTATATTTGCCTGGCAGTCACGGTTAACCTCGCAGGCGGGCAGCCGGTCTCCATGGCTAACATGCGCGCGGTG
LysLysLeuGlnLysLeuIleAspGluLysGlyAlaGluAsnIleAlaTyrIleCysLeuAlaValThrValAsnLeuAlaGlyGlyGlnProValSerMetAlaAsnMetArgAlaVal

CGTGAACTGACTGCAGCACACGGCATTAAAGTGTTCTACGACGCTACCCGCTGCGTAGAAAACGCCTACTTCATCAAAGAGCAAGAGCAGGGCTTTGAGAATAAGAGCATGGCCGAGATC
ArgGluLeuThrAlaAlaHisGlyIleLysValPheTyrAspAlaThrArgCysValGluAsnAlaTyrPheIleLysGluGlnGluGlnGlyPheGluAsnLysSerIleAlaGluIle

GTGCATGAGATGTTCAGCTACGCCGACGGTTGTACTATGAGTGGTAAAAAAGATTGTCTGGTGAATATCGGCGGCTTCCTGTGCATGAACGATGACGAAATGTTCTCTTCTGCCCAAAGAG
ValHisGluMetPheSerTyrAlaAspGlyCysThrMetSerGlyLysLysAspCysLeuValAsnIleGlyGlyPheLeuCysMetAsnAspAspGluMetPheSerSerAlaLysGlu

TTAGTCGTTGTCTACGAAGGCATGCCATCTTACGGCGGCCTGGCCGGACGCGACATGGAAGCCATGGCGATTGGTCTGCGCGAAGCCATGCAGTATGAGTACATCGAGCACCGCCGTGAAG
LeuValValValTyrGluGlyMetProSerTyrGlyGlyLeuAlaGlyArgAspMetGluAlaMetAlaIleGlyLeuArgGluAlaMetGlnTyrGluTyrIleGluHisArgValLys

FIG. 3-(2)

1080
CAGGTTCGCTATCTGGGCGACAAGCTGAAAGCCGCAGGTGTACCGATTGTTGAACCGGTGGGCCGGTCACGCCGGTATTCCTTGATGCGCCGTCGCTTCTGCCGAGCATCTGACGCAGGACGAG
GlnValArgTyrLeuGlyAspLysLeuLysAlaAlaGlyValProIleValGluProValGlyGlyHisAlaValPheLeuAspAlaArgArgPheCysGluHisLeuThrGlnAspGlu

1200
TTCCCGGCGCAAAGCCTGGCTGCCAGTATCTATGTGGAAACCGGCGTACGTAGTATGGAGCGCGGAATTATCTCTGCGGGCCGTAATAACGTGACCGGTGAACACCACAGGCCGAAACTG
PheProAlaGlnSerLeuAlaAlaSerIleTyrValGluThrGlyValArgSerMetGluArgGlyIleIleSerAlaGlyArgAsnAsnValThrGlyGluHisHisArgProLysLeu

1320
GAAACCGTGCCGTCTGACTATTCCACGCCGCGTTTATACTTACGCGCATATGGATGTGGTGGCTGACGGTATTATTAAACTTTACCAGCACAAAGAAGATATTCGCGGGCTGAAGTTTATT
GluThrValArgLeuThrIleProArgArgValTyrThrTyrAlaHisMetAspValValAlaAspGlyIleIleLysLeuTyrGlnHisLysGluAspIleArgGlyLeuLysPheIle

1368                                                                                                    1440
TACGAGCCGAAGCAGCTCCGTTTCTTTACTGCACGCTTTGACTATATCTAAATAATAATTATGGCCCCATCTCAGGATGGGGCTTTTTTGTATTTCTTTTCCATGAACAGGAAGCCTTTG
TyrGluProLysGlnLeuArgPhePheThrAlaArgPheAspTyrIle

1560
CGGTGCCCCGTTACCGTGACATAACTACAAAAGTAAACCCTGACTCCGACATTTTTATTATCCGCATGATGTACAAATGACAGCTGTAATTATAACGAATAGTCTACAGGAACATTTATCA

Nru I
TGGATATTAATGCCATTGAGCCGACAGAGTAGTACCCCCGGACTGATTAGCGGAACCATGTTGGTTATCGCGA

EP 0 394 479 A1

FIG. 4

EP 0 394 479 A1

# INTERNATIONAL SEARCH REPORT

International Application No PCT/JP89/01028

## I. CLASSIFICATION OF SUBJECT MATTER (if several classification symbols apply, indicate all) [6]

According to International Patent Classification (IPC) or to both National Classification and IPC

Int. Cl[5]　C12N15/60, 1/21, 9/88//(C12N15/60, C12R1:01),
(C12N1/21, C12R1:19),(C12N9/88, C12R1:19)

## II. FIELDS SEARCHED

| Minimum Documentation Searched [7] | |
|---|---|
| Classification System ı | Classification Symbols |
| IPC | C12N15/60, 1/21, 9/88 |

| Documentation Searched other than Minimum Documentation to the Extent that such Documents are Included in the Fields Searched [8] |
|---|
| Biological Abstracts Data Base (BIOSIS), Gen Bank Data Base, NBRF Protein Sequence Data Base |

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | JP, A, 63-222682 (Ajinomoto Co., Inc.), 16 September 1988 (16. 09. 88), (Family: none) | 1 - 9 |
| A | JP, A, 62-259590 (Idemitsu Kosan Co., Ltd.), 11 November 1987 (11. 11. 87), (Family: none) | 1 - 9 |
| A | Proc. Natl. Acad. Sci. USA, Vol. 84, No. 21, (1987) Byoung S. Kwon et al. [ Isolation and sequence of a c DNA clone for human tyrosinase that maps at the mouse c-albino locus ] P. 7473 - 7477 | 1 - 9 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance, the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| December 26, 1989 (26. 12. 89) | January 16, 1990 (16. 01. 90) |
| International Searching Authority | Signature of Authorized Officer |
| Japanese Patent Office | |

Form PCT/ISA/210 (second sheet) (January 1985)